(19) Europäisches Patentamt
European Patent Office
Office européen des brevets

(11) EP 0 810 213 B1

(12) EUROPEAN PATENT SPECIFICATION

(45) Date of publication and mention of the grant of the patent:
**06.09.2000 Bulletin 2000/36**

(51) Int Cl.[7]: **C07C 409/34**, C07C 409/32, C07C 407/00, C07C 409/00

(21) Application number: **97108629.3**

(22) Date of filing: **28.05.1997**

# (54) Organic peroxide stabilization with beta-dicarbonyl or cyclic alpha-diketone compounds

Stabilisierung von organischen Peroxiden mit Beta-dicarbonyl oder Alpha-diketon Verbindungen

Stabilisation de peroxides organiques par des composés beta-dicarbonyl ou alpha-diketone

(84) Designated Contracting States:
**BE DE FR GB IT LU NL**

(30) Priority: **31.05.1996 US 656093**
**31.05.1996 US 656094**

(43) Date of publication of application:
**03.12.1997 Bulletin 1997/49**

(73) Proprietor: **CK WITCO CORPORATION**
**Greenwich Connecticut 06831-2559 (US)**

(72) Inventors:
- **Frenkel, Peter**
  **Longview, Texas 75605 (US)**
- **Abma, Charles**
  **Marshall, Texas 75670 (US)**
- **Bock, Lawrence**
  **Longview, Texas 75604 (US)**
- **Andrews, Anthony**
  **Longview, Texas 75604 (US)**
- **Wells, Michael**
  **Longview, Texas 75601 (US)**

(74) Representative:
**Körber, Wolfhart, Dr. rer.nat. et al**
**Patentanwälte**
**Mitscherlich & Partner,**
**Sonnenstrasse 33**
**80331 München (DE)**

(56) References cited:
**DE-A- 1 593 704**
**US-A- 2 454 254**
**US-A- 3 869 401**
**US-A- 4 515 929**
**US-A- 5 334 326**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to organic peroxide compositions, and more specifically to peroxydicarbonate and diacyl peroxide compositions, in which a β-dicarbonyl compound or a cyclic α-diketone compound has been added to retard the rate of decomposition of the peroxide compound.

Organic peroxides, such as peroxydicarbonates and diacyl peroxides, are useful as free-radical initiators in the polymerization or copolymerization of ethylenically unsaturated monomers.

For example, organic peroxides are used as initiators in the polymerization of vinyl halides, such as vinyl chloride or vinyl bromide; vinylidene halides such as vinylidene chloride; and other compounds containing polymerizable unsaturated units. The products of this well known polymerization process have extensive commercial applications.

The polymerization of vinyl halides or the copolymerization of vinyl halides with vinylidene halides is usually conducted in an aqueous medium, i.e., emulsion, solution or suspension polymerization. In such polymerizations, the monomer or mixture of monomers is dispersed in water in the presence of a surfactant and thereafter the polymerization initiated with an organic peroxide. This is a well known reaction that has been widely reported.

All organic peroxides are by their nature hazardous materials. Their usefulness depends on their ability to decompose into free radicals, shown by the following reaction:

$$RO\text{-}OR' \rightarrow RO^{\cdot} + R'O^{\cdot}$$

The rate of this decomposition reaction at any given temperature depends on the structure of R and R'.

The decomposition reaction is exothermic. If exothermic decomposition were to occur during production, storage, or shipment, when the peroxides are in a concentrated form, excess pressure development and/or fire or explosion could result. Consequently, many organic peroxides must be kept refrigerated.

There have been several reports in recent years of the retardation of the rate of decomposition of organic peroxides.

The Journal of The American Chemical Society, Volume 72, pages 1254 to 1263 (1950), discloses the use of, for example, ethyl acetoacetate, iodine, trinitrobenzene, acetanilide, nitromethane, phenol, hydrogen peroxide, and tetralin to retard the rate of decomposition of diisopropyl peroxydicarbonate.

U.S. Patent No. 4,515,929 (1985) reports aqueous dispersions of organic peroxides including peroxydicarbonates, which are stabilized against decomposition by the addition of diphenyl peroxydicarbonate or di(alkyl substituted) phenyl peroxydicarbonates.

U.S. Patent No. 4,552,682 (1985) discloses the use of phenolic antioxidants to retard the rate of degradation of aqueous organic peroxide dispersions. The use of phenolic antioxidants is undesirable because they result in discoloration.

U.S. Patent No. 5,155,192 (1992) discloses the use of organic hydroperoxides, e.g., tert-butyl hydroperoxide, to retard the rate of decomposition of peroxydicarbonates.

Research Disclosure, April, 1995, page 275, reports the thermal stabilization of dialkyl peroxydicarbonates using unsaturated nitriles or unsaturated acetylenic compounds.

The present invention relates to the use of certain non-peroxide compounds which are effective in retarding the decomposition of organic peroxides such as peroxydicarbonates and diacyl peroxides. Thus, one aspect of the present invention is a composition containing an organic peroxide compound selected from the group consisting of peroxydicarbonate and diacyl peroxide compounds and at least one β-dicarbonyl or one cyclic α-diketone compound which reduces the rate of decomposition of the peroxide. Another aspect of the present invention is the method of stabilizing a peroxydicarbonate or diacyl peroxide against decomposition, comprising adding thereto a β-dicarbonyl compound or a cyclic α-diketone compound in an amount effective to achieve said stabilization.

In particular, β-dicarbonyl compounds useful in the present invention include those of formulas I, II and III:

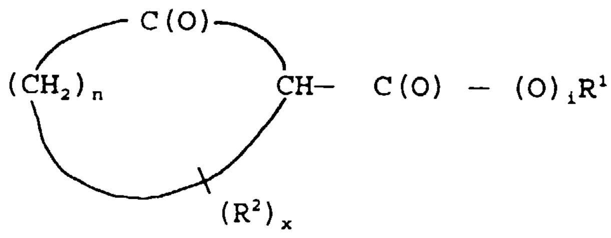

(I)

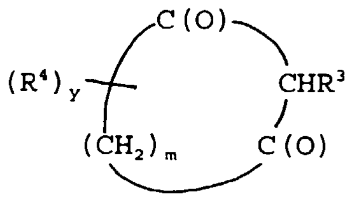

(II)

$$R^5\text{-C(O)-CHR}^6\text{-C(O)-R}^7 \qquad \text{(III)}$$

wherein

m is 1-6,
n is 1-6,
i is 0-1,
x is 0-2n,
y is 0-2m,
$R^1$ is alkyl containing 1 to 22 carbon atoms, phenyl, or phenyl substituted with one or more of alkyl containing 1 to 22 carbon atoms, halogen, and hydroxy, and $R^1$ can be hydrogen where i is zero,
$R^2$ is alkyl containing 1 to 22 carbon atoms, phenyl, or phenyl substituted with one or more of alkyl containing 1 to 22 carbon atoms, halogen, and hydroxy, and when x is greater than 1, each occurrence of $R^2$ can be the same or different and can be on the same or different ring carbon atoms;
$R^3$ is hydrogen, alkyl containing 1 to 22 carbon atoms, acyl containing 2 to 22 carbon atoms, phenyl, or phenyl substituted with one or more of alkyl containing 1 to 22 carbon atoms, halogen, and hydroxy;
$R^4$ is alkyl containing 1 to 22 carbon atoms, phenyl, or phenyl substituted with one or more of alkyl containing 1 to 22 carbon atoms, halogen, and hydroxy, and when y is greater than 1, each occurrence of $R^4$ can be the same or different and can be on the same or different ring carbon atoms;
$R^5$ is hydrogen, alkyl containing 1 to 22 carbon atoms, phenyl, or phenyl substituted with one or more of alkyl containing 1 to 22 carbon atoms, halogen, and hydroxy;
$R^6$ is hydrogen or alkyl containing 1 to 22 carbon atoms, phenyl or phenyl substituted with one or more of alkyl containing 1 to 22 carbon atoms, halogen, and hydroxy; or
$R^6$ is -C(O)OR$^8$ or -C(O)R$^8$ wherein $R^8$ is alkyl containing 1 to 22 carbon atoms; and
$R^7$ is phenyl or alkyl containing 1 to 22 carbon atoms.

Cyclic α-diketone compounds useful in the present invention include the formula (IV):

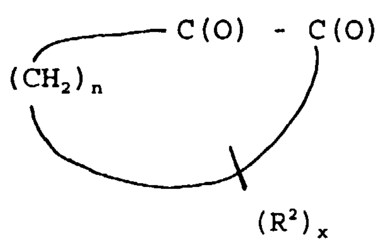

wherein n, x and $R^2$ are defined as above.

The present invention relates to compositions containing an organic peroxide, which is a peroxydicarbonate or a diacyl peroxide, and at least one β-dicarbonyl stabilizing compound or one cyclic α-diketone stabilizing compound to retard the rate of decomposition of the peroxide compound.

β-dicarbonyl compounds useful in the present invention may be of one of the following general formulas:

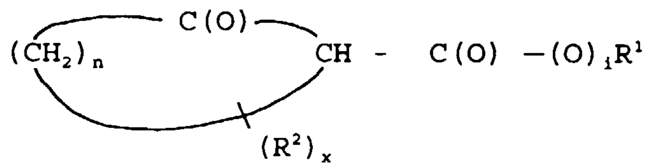

(I)

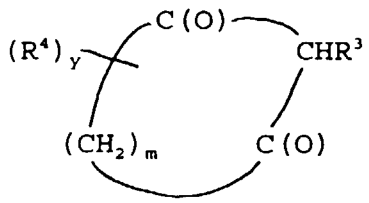

(II)

or

$$R^5 — C(O) — CHR^6 — C(O) — R^7 \qquad \text{(III)}$$

In Formula (I), n is 1-6 and preferably 3-5; x is zero up to 2n and i is 0-1; and $R^1$ is phenyl, substituted phenyl or alkyl containing 1 to 22 carbon atoms, and preferably 1 to 5 carbon atoms. The phrase "substituted phenyl" refers to phenyl substituted with alkyl containing 1 to 22 carbon atoms, halogen (i.e. fluorine, chlorine, bromine, and/or iodine), and/or hydroxy, or with any two or more of any such groups. That is, when two or more of such substituents are present they can be the same or different. The $R^2$ group can be phenyl, substituted phenyl or alkyl containing 1 to 22 carbon atoms and preferably 1 to 5 carbon atoms.

In Formula (II), m is 1-5 and preferably 2-4, y is zero up to 2m and $R^3$ can be hydrogen, phenyl or substituted phenyl. Alternatively, $R^3$ can be alkyl containing 1 to 22 carbon atoms and preferably 1 to 5 carbon atoms, or $R^3$ can be acyl containing 2 to 22 carbon atoms. The $R^4$ substituent can be phenyl, substituted phenyl, or alkyl containing 1 to 22 carbon atoms and preferably 1 to 5 carbon atoms.

In Formula (III), $R^5$ can be hydrogen, phenyl, substituted phenyl, or $R^5$ can be alkyl containing 1 to 22 carbon atoms and preferably 1 to 5 carbon atoms. $R^7$ can be phenyl, or $R^7$ can be alkyl containing 1 to 22 carbon atoms and preferably 1 to 5 carbon atoms. The $R^6$ group can be hydrogen, phenyl, substituted phenyl or can be alkyl containing 1 to 22 carbon atoms and preferably 1 to 5 carbon atoms. Also $R^6$ can be $-C(O)OR^8$ or $-C(O)R^8$; in these cases, $R^8$ is alkyl containing 1 to 22 carbon atoms and preferably 1 to 5 carbon atoms.

Cyclic $\alpha$-diketone compounds useful in the present invention are of the following general Formula (IV):

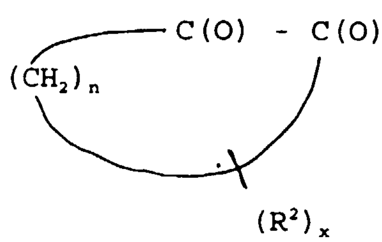

(IV)

In Formula (IV) n, x and $R^2$ are defined as above with respect to formula (I).

In all cases, alkyl substituents can be straight-chain; branched; cycloalkyl or cycloalkylalkyl. The cycloalkyl structure in the latter two cases may optionally be alkyl substituted.

Preferred embodiments useful in the present invention include cyclic ketone carboxylate compounds of Formula (I) such as ethyl-2-cyclopentanone carboxylate (wherein n is 3, i is 1, x is 0, and $R^1$ is ethyl), ethyl-2-cyclohexanone carboxylate (wherein n is 4, i is 1, x is 0, and $R^1$ is ethyl), methyl-2-cycloheptanone carboxylate (wherein n is 5, i is 1, x is 0, and $R^1$ is methyl), and ethyl-4-methyl-2-cyclohexanone-1-carboxylate (n is 4, i is 1, x is 1, $R^1$ is ethyl and $R^2$ is 4-methyl).

Other preferred embodiments of Formula (I) useful in the present invention include cyclic-β-diketones in which one of the carbonyl groups is contained in a cyclic structure, such as 2-acetyl cyclopentanone (wherein n is 3, i is 0, x is 0, and $R^1$ is methyl) and 2-acetyl cyclohexanone (n is 4, i is 0, x is 0, and $R^1$ is methyl).

Preferred embodiments useful in the present invention include compounds of Formula (II), such as 1, 3-cyclohexanedione (m is 3, y is 0, and $R^3$ is H) and 1, 3-cyclopentanedione (m is 2, y is 0, and $R^3$ is H).

Additional preferred embodiments useful in the present invention include compounds of Formula (III). Examples of such compounds include 2,4-pentanedione (wherein $R^5$ is methyl, $R^6$ is H, and $R^7$ is methyl) and dibenzoyl methane ($R^5$ and $R^7$ are phenyl, and $R^6$ is -H).

β-dicarbonyl compounds of Formulas (I), (II) or (III) are commercially available and/or can be synthesized from commercially available starting materials by use of procedures familiar to one of the ordinary skill in the art.

Still further preferred embodiments useful in the present invention include compounds of Formula (IV) such as 3-methyl-1, 2-cyclopetanedione (wherein n is 3, x is 1, and $R^2$ is 3-methyl); 3-ethyl-1, 2-cyclopentanedione (wherein n is 3, x is 1, and $R^2$ is 3-ethyl); 1,2-cyclohexanedione (wherein n is 4 and x is zero) and 1,2-cyclopentanedione (wherein n is 3 and x is zero).

Cyclic α-diketone compounds of Formula (IV) are commercially available and/or can be synthesized from commercially available starting materials by use of procedures familiar to one of ordinary skill in the art.

The amount of β-dicarbonyl or cyclic α-diketone to use in the compositions and methods of the present invention is an amount sufficient to retard the rate of decomposition of the peroxide compound. The preferred amount of β-dicarbonyl or cyclic α-diketone is a concentration of 0.2- 5.0% by weight of the peroxydicarbonate or diacyl peroxide present. The exact amount will vary and depend on both the peroxide compound and the β-dicarbonyl or cyclic α-diketone used, and on the conditions to which the peroxide composition is exposed.

The cyclic α-diketone can if desired be used in solution in an appropriate solvent. Suitable solvents for the cyclic α-diketone can be chosen from among alcohols, glycols and esters; an example is propylene glycol.

Peroxide compounds with which this invention is particularly useful are of the general structural formula (V):

$$R^9—(O)_c\text{-}C(O)—O—O—C(O)—(O)_c—R^{10} \qquad (V)$$

where each c is 0 or 1, and $R^9$ and $R^{10}$ can each be an aliphatic, cycloaliphatic or aromatic group with 1 to 22 carbon atoms, preferably 2 to 8 carbon atoms. When the subscripts c are zero, the compound is a diacyl peroxide, and when the subscripts c are one, the compound is a peroxydicarbonate. $R^9$ and $R^{10}$ may be branched or non-branched, substituted or unsubstituted alkyl, alkenyl, cycloalkyl or aromatic groups.

Examples of $R^9$ and $R^{10}$ groups include phenyl, methyl, ethyl, n-propyl, isopropyl, n-butyl, sec-butyl, t-butyl, isobutyl, hexyl, octyl, neopentyl, 2-ethylhexyl, capryl, lauryl, myristyl, cetyl, stearyl, allyl, methallyl, crotyl, cyclohexyl, 4-t-butylcyclohexyl, 4-t-amylcyclohexyl, benzyl, 2-phenylethyl, 2-phenylbutyl, α-carbethoxyethyl, β-methoxyethyl, 2-phenoxyethyl, 2-methoxyphenyl, 3-methoxyphenyl, 2-ethoxyethyl, 2-ethoxyphenyl, 3-methoxybutyl, 2-carbamyloxyethyl, 2-chloroethyl, 2-nitrobutyl and 2-nitro-2-methylpropyl.

Specific examples of peroxydicarbonates include diethyl peroxydicarbonate, di-n-butyl peroxydicarbonate, diisobutyl peroxydicarbonate, and di-4-tert-butylcyclohexyl peroxydicarbonate. Preferably the peroxydicarbonate is di-sec-butyl peroxydicarbonate, di-2-ethylhexyl peroxydicarbonate, di-n-propyl peroxydicarbonate or diisopropyl peroxydicarbonate.

Specific examples of diacyl peroxides include benzoyl peroxide, dilauroyl peroxide, didecanoyl peroxide, diacetyl peroxide, and di(3,5,5-trimethylhexanoyl) peroxide.

The peroxide compound may be symmetrical or unsymmetrical i.e., $R^9$ and $R^{10}$ may be the same or different. The peroxide may be a homogeneous mixture containing symmetric peroxides, asymmetric peroxides such as isopropyl-sec-butyl peroxydicarbonate or 2-methylpropionyl-3-methylpentanoyl peroxide or a mixture of symmetric and asymmetric peroxides such as mixtures of diisopropyl peroxydicarbonate, di-sec-butyl peroxydicarbonate and isopropyl-sec-butyl peroxydicarbonate as disclosed in U.S. Patent No. 4,269,726.

The peroxydicarbonate compounds and diacyl peroxide compounds can be synthesized by conventional techniques familiar to one of ordinary skill in the art. Peroxydicarbonates are typically prepared by reacting the corresponding alkyl chloroformate with aqueous sodium peroxide at low temperatures, 0°-20°C. See U.S. Patent No. 2,370,588 and the Journal of the American Chemical Society, Volume 72, page 1254 (1950). Diacyl peroxides are typically made from acid chlorides using synthetic techniques familiar to one of ordinary skill in the art.

Preferably, the peroxydicarbonates and diacyl peroxides with which this invention is useful include those which are a liquid at 0°C and more preferably a liquid at -5°C. Still more preferred are the peroxydicarbonates and diacyl peroxides which are liquid down to -20°C.

The present invention is especially applicable to aqueous dispersions of peroxydicarbonates and diacyl peroxides that are useful as initiators in the free radical polymerization of ethylenically unsaturated materials, particularly in an aqueous medium, e.g., suspension or emulsion polymerization. A dispersion of the peroxide is prepared by dispersing it in water with a suitable dispersing aid, e.g., a surfactant or emulsifying agent. Surfactants and emulsifying agents useful in the formulation of such dispersions are well known in this field and are quite numerous.

To prepare dispersions in accordance with the present invention, the β-dicarbonyl compound or cyclic α-diketone compound or its soluiton may be added to an already-formed peroxide dispersion, or to the water containing the surfactant, or to the peroxide before the dispersion is formed. Dispersions of the present invention generally contain 20 to 70% by weight, preferably 30 to 60% by weight, of the peroxydicarbonate compound or diacyl peroxide and 0.2 to 5% (by weight of the peroxide) of the β-dicarbonyl or cyclic α-diketone.

The manner of preparation of peroxide dispersions is known to one of ordinary skill in the art. A description of peroxydicarbonate dispersions and their preparation can be found in U.S. Patent No. 4,515,929; U.S. Patent No. 3,825,509; U.S. Patent No. 3,988,261 and U.S. Patent No. 4,092,470.

Peroxide compositions of the present invention may also be prepared as physical mixtures in the form of liquids, granules, powders or flakes. A physical mixture in accordance with the present invention may be prepared by mixing a liquid peroxide compound or a solution of a peroxide in a suitable solvent with the desired amount of β-dicarbonyl or cyclic α-diketone in a conventional mixing apparatus. The resulting mixture is then, if desired, granulated, pulverized or flaked. The β-dicarbonyl or cyclic α-diketone may be added either (1) to the chloroformate-or acid chloride-containing reaction mixture before preparation of the peroxide compound or (2) to the unprocessed reaction mixture immediately after the preparation of the peroxide compound. Either (1) or (2) will ensure that the two components are mixed as homogeneously as possible in order to receive the greatest possible benefit from the stabilizing effect of the β-dicarbonyl or cyclic α-diketone.

A solution of the present invention may be prepared by combining the desired amounts of β-dicarbonyl compound, or cyclic α-diketone compound or its solution, and peroxide in a suitable solvent.

Suitable organic solvents include those normally employed for peroxydicarbonate or diacyl peroxides, such as esters of phthalic acid, an example of which is dibutyl phthalate, and aliphatic and aromatic hydrocarbons and mixtures of such hydrocarbons, examples of which are hexane, odorless mineral spirits, mineral oil, benzene, toluene, xylene and (iso)paraffins such as isododecane. Other suitable solvents will be familiar to one of ordinary skill in the art.

Solutions according to the present invention preferably contain at least 10% by weight and more preferably at least 25% by weight of a peroxydicarbonate or diacyl peroxide compound.

The peroxide compositions of the present invention display numerous significant advantages. Chief among these is improved thermal stability, both in response to exposure to elevating temperature and in response to a given constant temperature. Thermal stability of self-reactive substances, in response to elevating temperatures, can be determined by measuring the self accelerating decomposition temperature (SADT). SADT is one of the recognized characteristics for determining the safe storage and transportation of materials such as organic peroxides. [Recommendations on the Transport of Dangerous Goods, 9th ed, United Nations, NY 1995, Section 11.3.5, page 264].

SADT can be directly correlated with the onset temperature as measured in a differential thermal analyzer (DTA). The onset temperature is the point at which an uncontrolled thermal decomposition starts. The onset temperature can be measured by determining the point at which the rate of temperature increase in a sealed cell exceeds a certain pre-determined value. In addition, the onset temperature can be measured by determining the point at which the rate of pressure increase in the sealed cell exceeds a certain pre-determined value.

Thermal stability in response to a given constant temperature can be assessed by means of accelerated aging tests at, for instance, 15°C.

The β-dicarbonyl and cyclic α-diketone compounds of the present invention increase the onset temperature of both peroxydicarbonates and diacyl peroxides.

Also, the β-dicarbonyl and cyclic α-diketone compounds do not detract from the effectiveness of the peroxide as a polymerization initiator.

**EXAMPLE 1**

The onset temperature was measured and compared for samples of pure di-2-ethylhexyl peroxydicarbonate and samples of di-2-ethylhexyl peroxydicarbonate in the presence of each of several different β-dicarbonyl compounds. The liquid mixtures were prepared by dissolving the required amount of β-dicarbonyl in the peroxydicarbonate.

Using a type of Differential Thermal Analyzer (Radex Solo Thermal Analyzer, marketed by Astra Scientific International, Pleasanton, CA), with an isothermal hold temperature of 30°C for 15 minutes and then a temperature increase of 1°/minute to 130°C, the onset temperature was measured for a one gram sample of di-2-ethylhexyl peroxydicarbonate in a sealed cell.

The onset temperature was measured both by noting the point where the rate of increase (ΔT) of the sample temperature reached 0.2°C/minute and also the point where the rate of increase in pressure (ΔP) of the closed sample

cell reached 6,895 kPa (1.0 psi)/minute. ΔT is the difference between the oven temperature and the sample temperature. ΔP is the difference between a reference pre-calibrated pressure and the pressure developed in the sealed sample cell.

The procedure was repeated with separate samples of the above peroxydicarbonate containing, in turn, ethyl-2-cyclohexanone carboxylate, 2-acetyl cyclohexanone, 2-acetyl cyclopentanone, and 2,4-pentanedione. The results are shown in Table I. Results obtained with ethyl acetoacetate, which is disclosed in the prior art, are included for comparison.

The results show that the presence of a β-dicarbonyl compound in accordance with the present invention increases the temperature at which self accelerating decomposition of the peroxydicarbonate will begin. This shows that the β-dicarbonyl compound is an effective stabilizer and is superior to ethyl acetocetate. The results also show that the effect is concentration dependent, with the decomposition beginning at a higher temperature when more β-dicarbonyl compound is present.

TABLE I

| ONSET TEMPERATURE FOR 98.3% DI-2-ETHYLHEXYL PEROXYDICARBONATE | | | |
|---|---|---|---|
| ADDITIVE | Additive Wt% | Onset Temperature (°C) | |
| | | by ΔT | by ΔP |
| None | -- | 37.3 | 40.2 |
| Ethyl acetoacetate | 2.9 | 43.9 | 46.9 |
| Ethyl-2-cyclohexanone carboxylate | 0.9 | 49.1 | 50.8 |
| Ethyl-2-cyclohexanone carboxylate | 2.9 | 55.2 | 57.0 |
| 2,4-Pentanedione | 2.9 | 55.3 | 58.2 |
| 2-Acetyl cyclohexanone | 2.9 | 55.6 | 57.5 |
| 2-Acetyl cyclopentanone | 3.1 | 57.7 | 61.2 |

**EXAMPLE 2**

The onset temperatures for samples of di-2-ethylhexyl peroxydicarbonate diluted with odorless mineral spirits (OMS) and samples of di-2-ethylhexyl peroxydicarbonate diluted in OMS in the presence of several different β-dicarbonyl compounds were measured and compared.

The liquid mixtures were prepared by dissolving the indicated amount of β-dicarbonyl compound in the peroxydicarbonate solution.

Using the same apparatus and procedure as described in Example 1, the onset temperature for a one gram sample of 82.5% di-2-ethylhexyl peroxydicarbonate diluted in OMS was measured. The procedure was repeated with separate samples of the above solution to which ethyl-2-cyclohexanone carboxylate, 2-acetyl cyclohexanone, 2-acetyl cyclopentanone, methyl-2-cycloheptanone carboxylate, ethyl-2-oxocyclopentane carboxylate, dibenzoyl methane, and ethyl-4-methyl-2-cyclohexanone-1-carboxylate had been added. The results are shown in Table II. Results obtained with ethyl acetoacetate, which is disclosed in the prior art, are included for comparison.

As can be seen in Table II, the addition of a β-dicarbonyl compound in accordance with the present invention increases the temperature at which self accelerating decomposition of the peroxydicarbonate will begin. The results also show that the effect is concentration dependent, with the decomposition of the peroxydicarbonate beginning at a higher temperature when more β-dicarbonyl compound is present.

TABLE II

| **ONSET TEMPERATURE FOR 82.5% DI-2-ETHYLHEXYL PEROXYDICARBONATE IN OMS** | | | |
|---|---|---|---|
| ADDITIVE | Additive Wt% | ONSET TEMPERATURE (°C) | |
| | | by ΔT | by ΔP |
| None | -- | 42.9 | 43.3 |
| Ethyl acetoacetate | 3.1 | 43.4 | 47.5 |
| Ethyl-2-cyclohexanone carboxylate | 0.2 | 43.1 | 46.0 |
| Ethyl-2-cyclohexanone carboxylate | 0.5 | 47.5 | 48.0 |
| Ethyl-2-cyclohexanone carboxylate | 1.0 | 50.0 | 51.6 |
| Ethyl-2-cyclohexanone carboxylate | 2.4 | 55.1 | 54.7 |

TABLE II (continued)

| ONSET TEMPERATURE FOR 82.5% DI-2-ETHYLHEXYL PEROXYDICARBONATE IN OMS | | | |
|---|---|---|---|
| ADDITIVE | Additive Wt% | ONSET TEMPERATURE (°C) | |
| | | by ΔT | by ΔP |
| Ethyl-2-cyclohexanone carboxylate | 5.0 | 58.6 | 57.4 |
| 2-Acetyl cyclohexanone | 1.0 | 51.9 | 51.5 |
| 2-Acetyl cyclohexanone | 1.9 | 54.8 | 56.7 |
| 2-Acetyl cyclohexanone | 3.0 | 57.5 | 57.1 |
| 2-Acetyl cyclopentanone | 1.0 | 54.0 | 55.3 |
| 2-Acetyl cyclopentanone | 1.9 | 57.4 | 57.9 |
| 2-Acetyl cyclopentanone | 2.8 | 58.4 | 59.0 |
| Methyl-2-cycloheptanone carboxylate | 1.1 | 44.7 | 47.0 |
| Ethyl-2-oxocyclopentane carboxylate | 1.1 | 47.2 | 47.2 |
| Dibenzoyl methane | 3.0 | 50.0 | 51.5 |
| Ethyl-4-methyl-2-cyclo-hexanone-1-carboxylate | 3.0 | 55.5 | 57.0 |

**EXAMPLE 3**

The onset temperatures for samples of di-sec-butyl peroxydicarbonate diluted in odorless mineral spirits (OMS) and samples of di-sec-butyl peroxydicarbonate diluted in OMS in the presence of several different (3-dicarbonyl compounds were measured and compared. The liquid mixtures were prepared by dissolving the indicated amount of β-dicarbonyl compound in the peroxydicarbonate solution. The onset temperature was measured according to the procedure described in Example I.

As can be seen in Table III, the addition of a β-dicarbonyl compound in accordance with the present invention increases the temperature at which self accelerating decomposition of the peroxydicarbonate will begin. The results also show that the effect is concentration dependent, with the reaction beginning at a higher temperature when more β-dicarbonyl compound is present. The effect of ethyl acetoacetate is included for comparison.

TABLE III

| ONSET TEMPERATURE FOR 75.5% DI-SEC-BUTYL PEROXYDICARBONATE IN OMS | | | |
|---|---|---|---|
| ADDITIVE | WT% | ONSET TEMPERATURE (°C) | |
| | | by ΔT | by ΔP |
| None | -- | 40.8 | 44.1 |
| Ethyl acetoacetate | 2.9 | 38.2 | 43.6 |
| Ethyl-2-cyclohexanone carboxylate | 1.1 | 46.8 | 47.3 |
| Ethyl-2-cyclohexanone carboxylate | 3.0 | 52.3 | 51.5 |
| 2-Acetyl cyclohexanone | 0.9 | 47.5 | 47.5 |
| 2-Acetyl cyclohexanone | 2.8 | 52.5 | 53.3 |
| 2-Acetyl cyclopentanone | 0.9 | 48.1 | 48.1 |
| 2-Acetyl cyclopentanone | 2.9 | 54.3 | 54.3 |
| 2,4-Pentanedione | 3.0 | 50.5 | 51.6 |

EXAMPLE 4

The effect of the presence of various β-dicarbonyl compounds on the storage stability at 15°C of pure di-2-ethylhexyl peroxydicarbonate was determined as an accelerated aging test.

The purity of the peroxydicarbonate was measured initially, after 7 days, and after 14 days. The results are presented in Table IV. Ethyl acetoacetate is included as an example of the prior art. The initial purity values were corrected for the presence of the additive.

A similar procedure was repeated with a sample of di-2-ethylhexyl peroxydicarbonate in OMS and a sample of di-sec-butyl peroxydicarbonate in OMS. The results are shown in Tables IV-A and IV-B, respectively. The initial purity values were corrected for the presence of the additive.

The results show that the presence of a β-dicarbonyl compound in accordance with the present invention retards the rate of decomposition of the peroxydicarbonate.

TABLE IV

| PURITY VS TIME AT 15°C FOR PURE DI-2-ETHYLHEXYL PEROXYDICARBONATE | | | | |
|---|---|---|---|---|
| ADDITIVE | Additive Wt% | Purity (%) | | |
| | | START | 7 DAYS | 14 DAYS |
| None | -- | 98.3 | 32.1 | 17.5 |
| Ethyl acetoacetate | 2.9 | 95.4 | 41.6 | 21.3 |
| Ethyl-2-cyclohexanone carboxylate | 1.1 | 97.3 | 70.4 | 30.4 |
| Ethyl-2-cyclohexanone carboxylate | 2.9 | 95.4 | 88.0 | 61.6 |
| 2-Acetyl cyclohexanone | 1.0 | 97.3 | 43.7 | n.d. |
| 2-Acetyl cyclohexanone | 2.9 | 95.2 | 58.8 | n.d. |
| 2-Acetyl cyclopentanone | 1.0 | 97.3 | 56.3 | n.d. |
| 2-Acetyl cyclopentanone | 3.0 | 95.4 | 71.2 | 43.3 |
| 2,4-Pentanedione | 2.9 | 95.4 | 78.1 | 57.2 |
| n.d.= not determined | | | | |

TABLE IV-A

| PURITY VS TIME AT 15°C FOR DI-2-ETHYLHEXYL PEROXYDICARBONATE IN OMS | | | | |
|---|---|---|---|---|
| ADDITIVE | Additive WT% | Purity (%) | | |
| | | START | 7 DAYS | 14 DAYS |
| None | -- | 76.2 | 33.8 | 24.9 |
| Ethyl-2-cyclohexanone carboxylate | 3.0 | 73.9 | 67.3 | 37.6 |
| 2-Acetyl cyclohexanone | 2.9 | 74.0 | 57.4 | 28.3 |
| 2-Acetyl cyclopentanone | 2.9 | 73.9 | 63.6 | 44.2 |
| 2,4-Pentanedione | 3.0 | 73.9 | 61.0 | 44.9 |

TABLE IV-B

| PURITY VS TIME AT 15°C FOR DI-SEC-BUTYL PEROXYDICARBONATE IN OMS | | | |
|---|---|---|---|
| ADDITIVE | Purity (%) | | |
| | Additive Wt% | START | 7 DAYS |
| None | -- | 75.5 | 49.6 |
| Ethyl-2-cyclohexanone carboxylate | 3.1 | 73.2 | 60.8 |
| 2-Acetyl cyclohexanone | 3.2 | 73.1 | 53.3 |
| 2-Acetyl cyclopentanone | 2.8 | 73.5 | 56.9 |

**EXAMPLE 5**

The onset temperatures for a sample of di-(3,5,5-trimethylhexanoyl) peroxide and samples of di-(3,5,5-trimethylhexanoyl) peroxide in the presence of several different β-dicarbonyl compounds were measured and compared. The liquid mixtures were prepared by dissolving the indicated amount of β-dicarbonyl compound in the peroxide.

Using the procedure described in Example 1, the onset temperature for a sample of 99% di-(3,5,5-trimethylhexanoyl) peroxide was measured. The procedure was repeated with separate samples of the above product to which 2,4-pentanedione and ethyl-2-cyclohexanone carboxylate had been added. The results are shown in Table V.

As can been seen in Table V, the addition of β-dicarbonyl compound in accordance with the present invention increases the temperature at which self accelerating decomposition of the diacyl peroxide will begin.

TABLE V

| ONSET TEMPERATURE FOR 99% DI(3,5,5-TRIMETHYLHEXANOYL) PEROXIDE | | | |
|---|---|---|---|
| ADDITIVE | Additive WT% | Onset Temperature (°C) | |
| | | by ΔT | by ΔP |
| None | --- | 68.2 | 67.7 |
| 2,4-pentanedione | 3.1 | 68.4 | 70.9 |
| Ethyl-2-cyclohexanone carboxylate | 3.0 | 69.8 | 68.7 |

**EXAMPLE 6**

The onset temperatures for a sample of di-(3,5,5-trimethylhexanoyl) peroxide diluted in odorless mineral spirits (OMS) and samples of di-(3,5,5-trimethylhexanoyl) peroxide in OMS in the presence of several different β-dicarbonyl compounds were measured and compared. The liquid mixtures were prepared by dissolving the indicated amount of β-dicarbonyl compound in the peroxide solution.

Using the procedure described in Example 1, the onset temperature for a sample of 60% di-(3,5,5-trimethylhexanoyl) peroxide in OMS was measured. The procedure was repeated with separate samples of the above solution to which 2,4-pentanedione and ethyl-2-cyclohexanone carboxylate had been added. The results are shown in Table VI.

As can been seen in Table VI, the addition of β-dicarbonyl compound in accordance with the present invention increases the temperature at which self accelerating decomposition of the diacyl peroxide solution will begin.

TABLE VI

| ONSET TEMPERATURE FOR 60% DI(3,5,5-TRIMETHYLHEXANOYL) PEROXIDE IN OMS | | | |
|---|---|---|---|
| ADDITIVE | Additive WT% | Onset Temperature (°C) | |
| | | by ΔT | by ΔP |
| None | --- | 74.9 | 76.1 |
| 2,4-pentanedione | 3.0 | 76.3 | 78.1 |
| Ethyl-2-cyclohexanone carboxylate | 3.0 | 76.4 | 77.5 |

**EXAMPLE 7**

The onset temperature was measured and compared for samples of pure di-2-ethylhexyl peroxydicarbonate and samples of di-2-ethylhexyl peroxydicarbonate in the presence of each of several different cyclic α-diketone compounds. The liquid mixtures were prepared by dissolving into the peroxydicarbonate a sufficient amount of a soluiton of the α-diketone in propylene glycol to provide the indicated amount of the α-diketone.

Using a type of Differential Thermal Analyzer (Radex Solo Thermal Analyzer, marketed by Astra Scientific International, Pleasanton, CA), with an isothermal hold temperature of 30°C for 15 minutes and then a temperature increase of 1°C minute to 130°C, the onset temperature was measured for a one gram sample of di-2-ethylhexyl peroxydicarbonate in a sealed cell.

The onset temperature was measured both by noting the point where the rate of increase (ΔT) of the sample temperature reached 0.2°C/minute and also the point where the rate of increase in pressure (ΔP) of the closed sample cell reached 6,895 kPa (1,0 psi) minute. ΔT is the difference between the oven temperature and the sample temperature. ΔP is the difference between a reference pre-calibrated pressure and the pressure developed in the sealed sample cell.

The procedure was repeated with separate samples of the above peroxydicarbonate containing, in turn, solutions (in propylene glycol) of 3-methyl-1,2-cyclopentanedione (MCPD), 3-ethyl-1,2-cyclopentanedione (ECPD) and 1,2-cyclohexanedione (CHD). The results are shown in Table VII. Results obtained with ethyl acetoacetate, which is disclosed in the prior art, are included for comparison.

The results show that the presence of a compound in accordance with the present invention increases the temperature at which self accelerating decomposition of the peroxydicarbonate will begin. This shows that the cyclic α-diketone compound is an effective stabilizer and is superior to ethyl acetocetate. The results also show that the effect is concentration dependent, with the decomposition beginning at a higher temperature when more cyclic α-diketone compound is present.

TABLE VII

| ONSET TEMPERATURE FOR PURE DI-2-ETHYLHEXYL PEROXYDICARBONATE | | | | |
|---|---|---|---|---|
| Sample Purity, % | Additive | Wt.% of Pure Additive Used | Onset Temperature (°C) | |
| | | | by ΔT | by ΔP |
| 97.7 | None | -- | 36.3 | 42.3 |
| | ECPD-50** | 1.8 | 54.8 | 57.2 |
| 97.4% | None | | 34.4 | 38.8 |
| | Ethylacetoacetate | 3.0 | 43.4 | 46.3 |
| | MCPD-10* | 0.3 | 42.8 | 45.7 |
| | MCPD-10 | 0.5 | 48.4 | 50.1 |
| | CHD-60*** | 0.6 | 42.3 | 44.9 |
| | CHD-60 | 1.8 | 48.4 | 49.0 |
| | CHD-60 | 3.0 | 51.3 | 52.1 |

* MCPD-10= 10% solution of MCPD in propylene glycol
** ECPD-50= 50% solution of ECPD in propylene glycol
*** CHD-60 = 60% solution of CHD in propylene glycol

## EXAMPLE 8

The onset temperatures for samples of di-2-ethylhexyl peroxydicarbonate diluted with odorless mineral spirits (OMS) and samples of di-2-ethylhexyl peroxydicarbonate diluted in OMS in the presence of several different cyclic α-diketone compounds were measured and compared.

The liquid mixtures were prepared by dissolving into the peroxydicarbonate solution a sufficient amount of a solution of ECPD, MCPD or CHD in propylene glycol, to provide the indicated amount of the cyclic α-diketone compound.

Using the same apparatus and procedure as described in Example 7, the onset temperature for a one gram sample of di-2-ethylhexyl peroxydicarbonate diluted in OMS was measured. The procedure was repeated with separate samples of the above solution to which a solution of cyclic α-diketone had been added. The results are shown in Table VIII. Results obtained with ethyl acetoacetate, which is disclosed in the prior art, are included for comparison. As can be seen in Table VIII, the addition of a cyclic α-diketone compound in accordance with the present invention increases the temperature at which self accelerating decomposition of the peroxydicarbonate solution will begin. The results also show that the effect is concentration dependent, with the decomposition beginning at a higher temperature when more cyclic α-diketone compound is present.

TABLE VIII

| ONSET TEMPERATURE FOR DI-2-ETHYLHEXYL PEROXYDICARBONATE IN OMS | | | | |
|---|---|---|---|---|
| Sample Purity, % | Additive | Wt.% of Pure Additive Used | Onset Temperature (°C) | |
| | | | by ΔT | by ΔP |
| 74.9 | None | | 41.4 | 43.6 |
| | ECPD-50* | 0.2 | 44.9 | 46.3 |
| | ECPD-50 | 0.4 | 48.5 | 48.5 |
| | ECPD-50 | 0.5 | 50.2 | 52.1 |
| | ECPD-50 | 0.9 | 54.1 | 54.8 |
| | ECPD-50 | 1.5 | 56.2 | 57.4 |
| | ECPD-50 | 2.5 | 57.0 | 58.9 |

* ECPD-50= 50% solution of ECPD in propylene glycol

TABLE VIII (continued)

| ONSET TEMPERATURE FOR DI-2-ETHYLHEXYL PEROXYDICARBONATE IN OMS | | | | |
|---|---|---|---|---|
| Sample Purity, % | Additive | Wt.% of Pure Additive Used | Onset Temperature (°C) | |
| | | | by ΔT | by ΔP |
| 75.1 | None | | 40.7 | 45.0 |
| | Ethylacetoacetate | 3.0 | 44.5 | 45.9 |
| | MCPD-10** | 0.1 | 41.6 | 45.3 |
| | MCPD-10 | 0.3 | 47.0 | 49.3 |
| | MCPD-10 | 0.5 | 48.6 | 50.4 |
| | CHD-60*** | 0.6 | 46.9 | 49.0 |
| | CHD-60 | 1.2 | 50.9 | 52.5 |
| | CHD-60 | 1.9 | 53.0 | 53.9 |

** MCPD-10= 10% solution of MCPD in propylene glycol

*** CHD-60 = 60% solution of CHD in propylene glycol

**EXAMPLE 9**

The onset temperatures for samples of di-sec-butyl peroxydicarbonate diluted in odorless mineral spirits (OMS) and samples of di-sec-butyl peroxydicarbonate diluted in OMS in the presence of several different cyclic α-diketone compounds were measured and compared. The liquid mixtures were prepared by dissolving into the peroxydicarbonate solution a sufficient amount of a solution of ECPD, MCPD or CHD in propylene glycol to provide the indicated amount of α-diketone compound. The onset temperature was measured according to the procedure described in Example 7.

As can be seen in Table IX, the presence of a cyclic α-diketone compound in accordance with the present invention increases the temperature at which self accelerating decomposition of the peroxydicarbonate solution will begin. The results also show that the effect is concentration dependent, with the reaction beginning at a higher temperature when more cyclic α-diketone compound is present. The effect of ethyl acetoacetate is included for comparison.

TABLE IX

| ONSET TEMPERATURE FOR DI-SEC-BUTYL PEROXYDICARBONATE IN OMS | | | | |
|---|---|---|---|---|
| Sample Purity, % | Additive | Wt.% of Pure Additive Used | Onset Temperature (°C) | |
| | | | by ΔT | by ΔP |
| 76.2 | None | -- | 36.6 | 41.0 |
| | ECPD-50* | 1.5 | 52.5 | 53.0 |
| 70.7 | None | | 37.0 | 37.9 |
| | Ethylacetoacetate | 3.0 | 36.3 | 39.3 |
| | MCPD-10** | 0.1 | 39.9 | 41.3 |
| | MCPD-10 | 0.3 | 44.2 | 46.7 |
| | MCPD-10 | 0.5 | 46.8 | 48.1 |
| | CHD-60*** | 0.6 | 43.6 | 45.0 |
| | CHD-60 | 1.8 | 50.0 | 50.7 |
| | CHD-60 | 3.0 | 52.8 | 53.8 |

* ECPD-50= 50% solution of ECPD in propylene glycol

** MCPD-10= 10% solution of MCPD in propylene glycol

*** CHD-60 = 60% solution of CHD in propylene glycol

EXAMPLE 10

The effect of the presence of ECPD on the storage stability at 15°C of pure di-2-ethylhexyl peroxydicarbonate, di-2-ethylhexyl peroxydicarbonate dissolved in odorless mineral spirits (OMS), and di-sec-butyl peroxydicarbonate dissolved in OMS was determined as an accelerated aging test. The results are presented in Table X.
The purity of the peroxydicarbonate was measured at the times indicated in Table X. The initial purity values in the Table were corrected for the presence of the additive.
The results show that the presence of a cyclic α-diketone, in accordance with the present invention, retards the rate of decomposition of the peroxydicarbonate.

Table X -

| **Purity vs. Time at 15°C for Several Peroxydicarbonates** (ECPD was added as a 50% solution in propylene glycol) | | | | | | |
|---|---|---|---|---|---|---|
| Peroxydicarbonate | Additive | Wt.% of Pure Additive Used | Purity (%) | | | |
| | | | Start | 7 Days | 14 Days | 21 Days |
| 97.7% Di-2-ethylhexyl Peroxydicarbonate (pure) | none | -- | 97.7 | 37.3 | 22.4 | 21.7 |
| " | ECPD-50 | 1.5 | 94.9 | 77.3 | 39.3 | 27.1 |
| 74.9% Di-2-ethylhexyl Peroxydicarbonate in OMS | none | -- | 74.9 | 28.6 | 17.9 | 15.4 |
| " | ECPD-50 | 1.5 | 72.7 | 58.8 | 37.7 | 23.1 |
| 76.2% Di-sec-butyl Peroxydicarbonate in OMS | none | -- | 76.2 | 19.9 | 17.7 | -- |
| " | ECPD-50 | 1.4 | 74.2 | 49.5 | 20.7 | -- |

**EXAMPLE 11**

The onset temperatures for a sample of pure di-(3,5,5-trimethylhexanoyl) di-(3,5,5-trimethylhexanoyl) peroxide and samples of pure peroxide in the presence of two different cyclic α-diketones were measured and compared. This procedure was repeated for a sample of di-(3,5,5-trimethylhexanoyl) peroxide dissolved in odorless mineral spirits (OMS) and samples of di-(3,5,5-trimethylhexanoyl) peroxide dissolved in OMS in the presence of two different cyclic α-diketone compounds.
The liquid mixtures were prepared by adding a sufficient amount of a solution of ECPD or CHD in propylene glycol to the diacyl peroxide to provide the indicated amount of the cyclic α-diketone compound. The procedure described in Example 7 was followed. The results are shown in Table XI.
As can be seen in Table XI, the presence of the cyclic α-diketone compound, in accordance with the present invention, increases the temperature at which self accelerating decomposition of the diacyl peroxide, or its solution in OMS, will begin.

TABLE XI

| **ONSET TEMPERATURE FOR DI(3,5,5-TRIMETHYLHEXANOYL) PEROXIDE** | | | | |
|---|---|---|---|---|
| Sample Purity, % | Additive | Wt.% of pure Additive Used | Onset Temperature (°C) | |
| | | | by ΔT | by ΔP |
| 98.2 | None | | 68.2 | 67.7 |
| | CHD-60* | 3.0 | 70.1 | 72.3 |
| | ECPD-50** | 2.6 | 70.8 | 74.4 |

* CHD-60 = 60% solution of CHD in propylene glycol
** ECPD-50= 50% solution of ECPD in propylene glycol

TABLE XI (continued)

| ONSET TEMPERATURE FOR DI(3,5,5-TRIMETHYLHEXANOYL) PEROXIDE | | | | |
|---|---|---|---|---|
| Sample Purity, % | Additive | Wt.% of pure Additive Used | Onset Temperature (°C) | |
| | | | by ΔT | by ΔP |
| 60.1(in OMS) | None | | 74.9 | 76.1 |
| | CHD-60* | 2.0 | 76.6 | 76.6 |
| | ECPD-50** | 1.5 | 77.0 | 77.0 |

* CHD-60 = 60% solution of CHD in propylene glycol

** ECPD-50= 50% solution of ECPD in propylene glycol

## Claims

**1.** A composition comprising:

a. an organic peroxide component selected from the group consisting of peroxydicarbonate compounds, diacyl peroxides, and mixtures thereof; and

b. a sufficient amount of a stabilizer to retard the rate of decomposition of the organic peroxide component, wherein said stabilizer is selected from the group consisting of β-dicarbonyl compounds of formulas I, II and III and compounds of Formula IV:

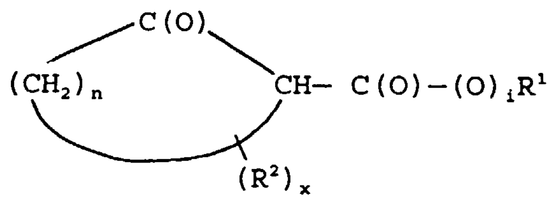

(I)

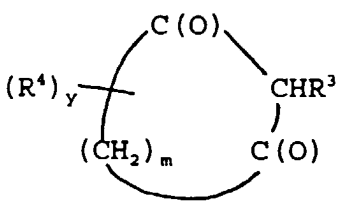

(II)

$$R^5\text{-}C(O)\text{-}CHR^6\text{-}C(O)\text{-}R^7 \qquad (III)$$

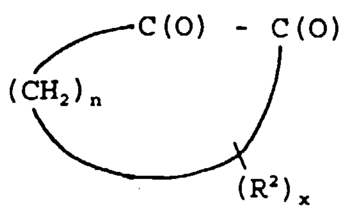

(IV)

and mixtures thereof, wherein m is 1-5,
n is 1-6,
i is 0-1,
x is 0-2n,

y is 0-2m,

$R^1$ is alkyl containing 1 to 22 carbon atoms, phenyl, or phenyl substituted with one or more of alkyl containing 1 to 22 carbon atoms, halogen, and hydroxy, and $R^1$ can be hydrogen where i is zero,
$R^2$ is alkyl containing 1 to 22 carbon atoms, phenyl, or phenyl substituted with one or more of alkyl containing 1 to 22 carbon atoms, halogen, and hydroxy, and when x is greater than 1, each occurrence of $R^2$ can be the same or different and can be on the same or different ring carbon atoms,
$R^3$ is hydrogen, alkyl containing 1 to 22 carbon atoms, acyl containing 2 to 22 carbon atoms, phenyl, or phenyl substituted with one or more of alkyl containing 1 to 22 carbon atoms, halogen, and hydroxy;
$R^4$ is alkyl containing 1 to 22 carbon atoms, phenyl, or phenyl substituted with one or more of alkyl containing 1 to 22 carbon atoms, halogen, and hydroxy; and when y is greater than 1, each occurrence of $R^4$ can be the same or different and can be on the same or different ring carbon atoms;
$R^5$ is hydrogen, alkyl containing 1 to 22 carbon atoms, phenyl, or phenyl substituted with one or more of alkyl containing 1 to 22 carbon atoms, halogen, or hydroxy;
$R^6$ is hydrogen or alkyl containing 1 to 22 carbon atoms, phenyl, or phenyl substituted with one or more of alkyl containing 1 to 22 carbon atoms, halogen, or hydroxy; or $R^6$ can be -C(O)O$R^8$ or -C(O)$R^8$ wherein $R^8$ is alkyl containing 1 to 22 carbon atoms; and
$R^7$ is phenyl or alkyl containing 1 to 22 carbon atoms.

**2.** A composition according to claim 1 comprising a β-dicarbonyl compound of formula (I).

**3.** A composition according to claim 1 comprising a β-dicarbonyl compound of formula (II).

**4.** A composition according to claim 1 comprising a β-dicarbonyl compound of formula (III).

**5.** A composition according to claim 1 comprising a compound of formula (IV).

**6.** A composition according to anyone of claims 1 to 5 wherein said organic peroxide component comprises at least one compound of the formula (V)

$$R^9\text{-(O)}_c\text{-C(O)-O-O-C(O)-(O)}_c\text{-}R^{10} \qquad (V)$$

wherein $R^9$ and $R^{10}$ are independently aliphatic, cycloaliphatic or aromatic groups containing 1 to 22 carbon atoms, and c is zero or one.

**7.** A composition according to claim 6 wherein in formula (V) both subscripts c are one.

**8.** A composition according to claim 6 wherein in formula (V) both subscripts c are zero.

**9.** A composition according to claim 6 wherein $R^9$ and $R^{10}$ are independently selected from the group consisting of phenyl, methyl, ethyl, n-propyl, isopropyl, n-butyl, sec-butyl, t-butyl, isobutyl, hexyl, octyl, neopentyl, 2-ethylhexyl, capryl, lauryl, myristyl, cetyl, stearyl, allyl, methallyl, crotyl, cyclohexyl, 4-t-butylcyclohexyl, 4-t-amylcyclohexyl, benzyl, 2-phenylethyl, 2-phenylbutyl, α-carbethoxyethyl, β-methoxyethyl, 2-phenoxyethyl, 2-methoxyphenyl, 3-methoxyphenyl, 2-ethoxyethyl, 2-ethoxyphenyl, 3-methoxybutyl, 2-carbamyloxyethyl, 2-chloroethyl, 2-nitrobutyl and 2-nitro-2-methylpropyl.

**10.** A composition according to claim 1 wherein said stabilizer is selected from the group consisting of ethyl-2-cyclopentanone carboxylate, methyl-2-cycloheptanone carboxylate, ethyl-2-cyclohexanone carboxylate, 2-acetyl cyclopentanone, 2-acetyl cyclohexanone, ethyl-4-methyl-2-cyclohexanone-1-carboxylate, 1,3-cyclohexanedione, 1,3-cyclopentanedione, 2,4-pentanedione and dibenzoyl methane, 3-methyl-1, 2-cyclopentanedione, 3-ethyl-1, 2-cyclopentanedione, 1,2-cyclohexanedione, 1-2-cyclopentanedione and mixtures thereof.

**11.** A composition according to claim 1 wherein said stabilizer comprises 0.2 to 5% by weight of said organic peroxide component.

**12.** A composition according to claim 1 wherein said organic peroxide component is selected from the group consisting

of di-2-ethylhexyl peroxydicarbonate, di-sec-butyl peroxydicarbonate, diethyl peroxydicarbonate, di-n-butyl peroxydicarbonate, isopropyl-sec-butyl peroxydicarbonate, di-4-tert-butylcyclohexyl peroxydicarbonate, di-n-propyl peroxydicarbonate, diisopropyl peroxydicarbonate, benzoyl peroxide, dilauroyl peroxide, didecanoyl peroxide, diacetyl peroxide, 2-methylpropionyl-3-methylpentanoyl peroxide, and di-(3,5,5-trimethylhexanoyl) peroxide, and mixtures thereof.

**13.** The method of retarding the rate of decomposition of an organic peroxide selected from the group consisting of peroxydicarbonate and diacyl peroxide compounds and mixtures thereof comprising adding to said organic peroxide a stabilizer in an amount thereof effective to retard the rate of said decomposition, wherein said stabilizer is selected from the group consisting of β-dicarbonyl compounds of formulas I, II and III and compounds of formula IV:

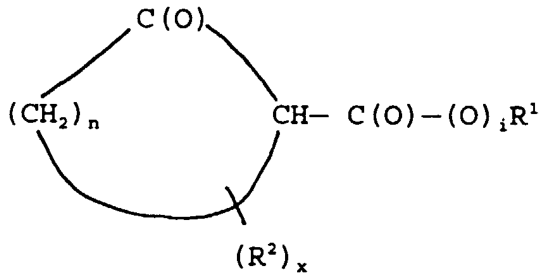

(I)

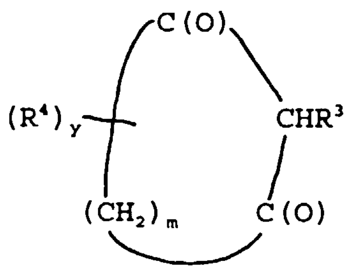

(II)

$$R^5\text{-C(O)-CHR}^6\text{-C(O)-R}^7 \qquad \text{(III)}$$

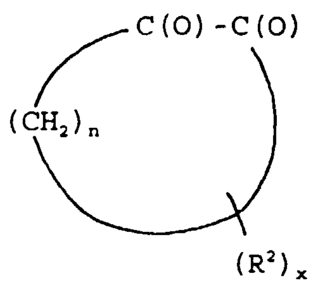

(IV)

and mixtures thereof, wherein m is 1-5,

n is 1-6,
i is 0-1,
x is 0-2n,
y is 0-2m,

$R^1$ is alkyl containing 1 to 22 carbon atoms, phenyl, or phenyl substituted with one or more of alkyl containing 1 to 22 carbon atoms, halogen and hydroxy, and $R^1$ can be hydrogen where i is zero,

$R^2$ is alkyl containing 1 to 22 carbon atoms, phenyl, or phenyl substituted with one or more of alkyl containing 1 to 22 carbon atoms, halogen and hydroxy, and when x is greater than 1, each occurrence of $R^2$ can be the same or different and can be on the same or different ring carbon atoms,
$R^3$ is hydrogen, alkyl containing 1 to 22 carbon atoms, acyl containing 2 to 22 carbon atoms, phenyl, or phenyl substituted with one or more of alkyl containing 1 to 22 carbon atoms, halogen, and hydroxy;
$R^4$ is alkyl containing 1 to 22 carbon atoms, phenyl, or phenyl substituted with one or more of alkyl containing 1 to 22 carbon atoms, halogen and hydroxy, and when y is greater than 1, each occurrence of $R^4$ can be the same or different and can be on the same or different ring carbon atoms;
$R^5$ is hydrogen, alkyl containing 1 to 22 carbon atoms, phenyl, or phenyl substituted with one or more of alkyl containing 1 to 22 carbon atoms, halogen, and hydroxy;
$R^6$ is hydrogen or alkyl containing 1 to 22 carbon atoms, phenyl, or phenyl substituted with one or more of alkyl containing 1 to 22 carbon atoms, halogen, and hydroxy; or $R^6$ can be -C(O)OR$^8$ or -C(O)R$^8$ wherein $R^8$ is alkyl containing 1 to 22 carbon atoms; and
$R^7$ is phenyl or alkyl containing 1 to 22 carbon atoms.

**14.** A method according to claim 13 wherein said peroxydicarbonate and diacyl peroxide compounds correspond to formula (V):

$$R^9\text{-(O)}_c\text{-C(O)-O-O-C(O)-(O)}_c\text{-}R^{10} \qquad (V)$$

wherein $R^9$ and $R^{10}$ are independently aliphatic, cycloaliphatic or aromatic groups containing 1 to 22 carbon atoms, and c is zero or one.

**15.** A method according to claim 14 wherein in formula (V) both subscripts c are one.

**16.** A method according to claim 14 wherein in formula (V) both subscripts c are zero.

**17.** A method according to claim 14 wherein $R^9$ and $R^{10}$ are independently selected from the group consisting of phenyl, methyl, ethyl, n-propyl, isopropyl, n-butyl, sec-butyl, t-butyl, isobutyl, hexyl, octyl, neopentyl, 2-ethylhexyl, capryl, lauryl, myristyl, cetyl, stearyl, allyl, methallyl, crotyl, cyclohexyl, 4-t-butylcyclohexyl, 4-t-amylcyclohexyl, benzyl, 2-phenylethyl, 2-phenylbutyl, α-carbethoxyethyl, β-methoxyethyl, 2-phenoxyethyl, 2-methoxyphenyl, 3-methoxyphenyl, 2-ethoxyethyl, 2-ethoxyphenyl, 3-methoxybutyl, 2-carbamyloxyethyl, 2-chloroethyl, 2-nitrobutyl and 2-nitro-2-methylpropyl.

**18.** A method according to claim 13 wherein said stabilizer is selected from the group consisting of ethyl-2-cyclopentanone carboxylate, methyl-2-cycloheptanone carboxylate, ethyl-2-cyclohexanone carboxylate, 2-acetyl cyclopentanone, 2-acetyl cyclohexanone, ethyl-4-methyl-2-cyclohexanone-1-carboxylate, 1,3-cyclohexanedione, 1,3-cyclopentanedione, 2,4-pentanedione and dibenzoyl methane, 3-methyl-1, 2-cyclopentanedione, 3-ethyl-1,2-cyclopentanedione, 1-2-cyclohexanedione, 1,2-cyclopentanedione, and mixtures thereof.

**19.** A method according to claim 13 wherein the amount of said stabilizer is 0.2 to 5% by weight of said organic peroxide.

**20.** A method according to claim 13 wherein said stabilizer comprises a β-dicarbonyl compound of formula (I).

**21.** A method according to claim 13 wherein said stabilizer comprises a (3-dicarbonyl compound of formula (II).

**22.** A method according to claim 13 wherein said stabilizer comprises a β-dicarbonyl compound of formula (III).

**23.** A method according to claim 13 wherein said stabilizer comprises a compound of formula (IV).

**24.** A method according to claim 13 wherein said organic peroxide component is selected from the group consisting of di-2-ethylhexyl peroxydicarbonate, di-sec-butyl peroxydicarbonate, diethyl peroxydicarbonate, di-n-butyl peroxydicarbonate, isopropyl-sec-butyl peroxydicarbonate, di-4-tert-butylcyclohexyl peroxydicarbonate, di-n-propyl peroxydicarbonate, diisopropyl peroxydicarbonate, benzoyl peroxide, dilauroyl peroxide, didecanoyl peroxide, diacetyl peroxide, 2-methylpropionyl-3-methylpentanoyl peroxide, and di-(3,5,5-trimethylhexanoyl) peroxide and mixtures thereof.

## Patentansprüche

**1.** Zusammensetzung, die umfaßt:

a. eine organische Peroxidkomponente, die aus der Gruppe, die aus Peroxydicarbonatverbindungen, Diacylperoxiden und Mischungen davon besteht, ausgewählt wird; und

b. eine ausreichende Menge eines Stabilisators, um die Zerfallsgeschwindigkeit der organischen Peroxidverbindung zu verzögern, wobei der Stabilisator aus der Gruppe ausgewählt wird, die besteht aus β-Dicarbonylverbindungen gemäß den Formeln I, II und III und Verbindungen gemäß der Formel IV:

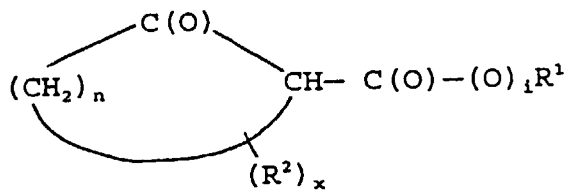

(I)

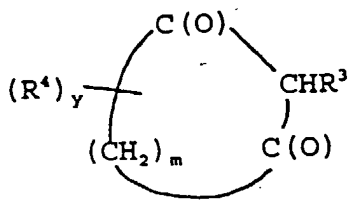

(II)

$$R^5\text{-C(O)-CHR}^6\text{-C(O)-R}^7 \qquad \text{(III)}$$

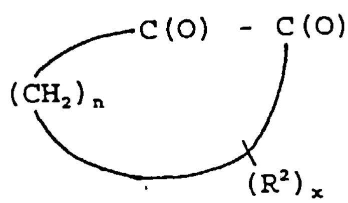

(IV)

und Mischungen davon, wobei

m 1-5 ist,
n 1-6 ist,
i 0-1 ist,
x 0-2n ist,
y 0-2m ist,
$R^1$ ist Alkyl, enthaltend 1 bis 22 Kohlenstoffatome, Phenyl oder Phenyl, substituiert mit einem oder mehreren Alkyl, enthaltend 1 bis 22 kohlenstoffatome, Halogen und Hydroxy, und $R^1$ kann Wasserstoff sein, wenn i Null ist,
$R^2$ ist Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen und Hydroxy, und wenn x größer als 1 ist, kann jedes Vorkommen von $R^2$ gleich oder verschieden sein und kann am gleichen oder an verschiedenen Ring-Kohlenstoffatomen vorliegen,
$R^3$ ist Wasserstoff, Alkyl, enthaltend 1 bis 22 Kohlenstoffatome, Acyl, enthaltend 2 bis 22 Kohlenstoffatome, Phenyl oder Phenyl, substituiert mit einem oder mehreren Alkyl, enthaltend 1 bis 22 Kohlenstoffatome, Halogen und Hydroxy;
$R^4$ ist Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen und Hydroxy; und wenn y größer als 1 ist,

kann jedes Vorkommen von $R^4$ gleich oder verschieden sein und kann am gleichen oder an verschiedenen Ring-Kohlenstoffatomen vorliegen;
$R^5$ ist Wasserstoff, Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen oder Hydroxy;
$R^6$ ist Wasserstoff oder Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen oder Hydroxy; oder $R^6$ kann -C(O)OR$^8$ oder -C(O)R$^8$ sein, wobei $R^8$ ist Alkyl, enthaltend 1 bis 22 Kohlenstoffatome; und
$R^7$ ist Phenyl oder Alkyl, enthaltend 1 bis 22 Kohlenstoffatome.

**2.** Zusammensetzung gemäß Anspruch 1, die eine β-Dicarbonylverbindung gemäß Formel (I) umfaßt.

**3.** Zusammensetzung gemäß Anspruch 1, die eine β-Dicarbonylverbindung gemäß Formel (II) umfaßt.

**4.** Zusammensetzung gemäß Anspruch 1, die eine β-Dicarbonylverbindung gemäß Formel (III) umfaßt.

**5.** Zusammensetzung gemäß Anspruch 1, die eine Verbindung gemäß Formel (IV) umfaßt.

**6.** Zusammensetzung gemäß einem der Ansprüche 1 bis 5, wobei die organische Peroxidverbindung wenigstens eine Verbindung gemäß der Formel (V)

$$R^9\text{-(O)}_c\text{-C(O)-O-O-C(O)-(O)}_c\text{-}R^{10} \qquad \text{(V)}$$

umfaßt, wobei $R^9$ und $R^{10}$ unabhängig voneinander aliphatische, cycloaliphatische oder aromatische Gruppen, enthaltend 1 bis 22 Kohlenstoffatome, sind, und c ist Null oder Eins.

**7.** Zusammensetzung gemäß Anspruch 6, wobei in Formel (V) beide Indizes c Eins sind.

**8.** Zusammensetzung gemäß Anspruch 6, wobei in Formel (V) beide Indizes c Null sind.

**9.** Zusammensetzung gemäß Anspruch 6, wobei $R^9$ und $R^{10}$ unabhängig voneinander aus der Gruppe, die aus Phenyl, Methyl, Ethyl, n-Propyl, Isopropyl, n-Butyl, sec-Butyl, t-Butyl, Isobutyl, Hexyl, Octyl, Neopentyl, 2-Ethylhexyl, Capryl, Lauryl, Myristyl, Cetyl, Stearyl, Allyl, Methallyl, Crotyl, Cyclohexyl, 4-t-Butylcyclohexyl, 4-t-Amylcyclohexyl, Benzyl, 2-Phenylethyl, 2-Phenylbutyl, α-Carbethoxyethyl, β-Methoxyethyl, 2-Phenoxyethyl, 2-Methoxyphenyl, 3-Methoxyphenyl, 2-Ethoxyethyl, 2-Ethoxyphenyl, 3-Methoxybutyl, 2-Carbamyloxyethyl, 2-Chlorethyl, 2-Nitrobutyl und 2-Nitro-2-methylpropyl besteht, ausgewählt wird.

**10.** Zusammensetzung gemäß Anspruch 1, wobei der Stabilisator aus der Gruppe, die aus Ethyl-2-cyclopentanoncarboxylat, Methyl-2-cycloheptanoncarboxylat, Ethyl-2-cyclohexanoncarboxylat, 2-Acetylcyclopentanon, 2-Acetylcyclohexanon, Ethyl-4-methyl-2-cyclohexanon-1-carboxylat, 1,3-Cyclohexandion, 1,3-Cyclopentandion, 2,4-Pentandion und Dibenzoylmethan, 3-Methyl-1,2-cyclopentandion, 3-Ethyl-1,2-cyclopentandion, 1,2-Cyclohexandion, 1-2-Cyclopentandion und Mischungen davon besteht, ausgewählt wird.

**11.** Zusammensetzung gemäß Anspruch 1, wobei der Stabilisator 0,2 bis 5 Gew.-% der organischen Peroxidkomponente umfaßt.

**12.** Zusammensetzung gemäß Anspruch 1, wobei die organische Peroxidkomponente aus der Gruppe, die aus Di-2-ethylhexylperoxydicarbonat, Di-sec-butyl-peroxydicarbonat, Diethylperoxydicarbonat, Di-n-butylperoxydicarbonat, Isopropyl-sec-butylperoxydicarbonat, Di-4-tert-butylcyclohexylperoxydicarbonat, Di-n-propylperoxydicarbonat,Diisopropylperoxydicarbonat, Benzoylperoxid, Dilauroylperoxid, Didecanoylperoxid, Diacetylperoxid, 2-Methylpropionyl-3-methylpentanoylperoxid und Di-(3,5,5-trimethylhexanoyl)peroxid und Mischungen davon besteht, ausgewählt wird.

**13.** Verfahren zum Verzögern der Zerfallsgeschwindigkeit eines organischen Peroxids, das aus der Gruppe, die aus Peroxydicarbonat- und Diacylperoxidverbindungen und Mischungen davon besteht, ausgewählt wird, das umfaßt ein Zugeben eines Stabilisators zu dem organischen Peroxid in einer Menge, die wirksam ist, um die Geschwindigkeit des Zerfalls zu verzögern, wobei der Stabilisator aus der Gruppe ausgewählt wird, die besteht aus β-Dicarbonylverbindungen gemäß den Formeln I, II und III und Verbindungen gemäß der Formel IV:

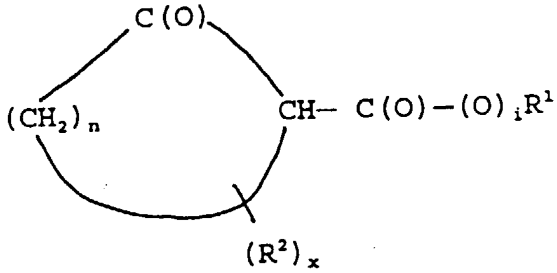

(I)

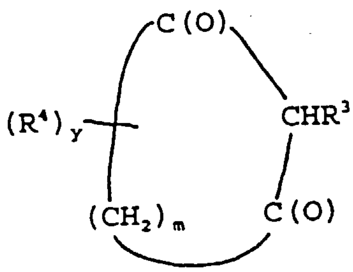

(II)

$$R^5\text{-C(O)-CHR}^6\text{-C(O) -R}^7 \qquad \text{(III)}$$

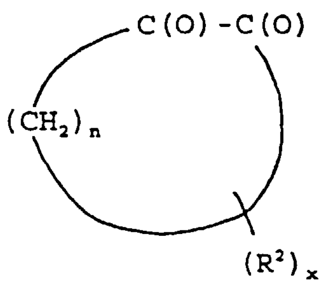

(IV)

und Mischungen davon, wobei

m 1-5 ist,
n 1-6 ist,
i 0-1 ist,
x 0-2n ist,
y 0-2m ist,
$R^1$ ist Alkyl, enthaltend 1 bis 22 Kohlenstoffatome, Phenyl oder Phenyl, substituiert mit einem oder mehrerer Alkyl, enthaltend 1 bis 22 Kohlenstoffatome, Halogen und Hydroxy, und $R^1$ kann Wasserstoff sein, wenn i Null ist,
$R^2$ ist Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen und Hydroxy, und wenn x größer als 1 ist, kann jedes Vorkommen von $R^2$ gleich oder verschieden sein und kann am gleichen oder an verschiedenen Ring-Kohlenstoffatomen vorliegen,
$R^3$ ist Wasserstoff, Alkyl, enthaltend 1 bis 22 Kohlenstoffatome, Acyl, enthaltend 2 bis 22 Kohlenstoffatome, Phenyl oder Phenyl, substituiert mit einem oder mehreren Alkyl, enthaltend 1 bis 22 Kohlenstoffatome, Halogen und Hydroxy;
$R^4$ ist Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen und Hydroxy; und wenn y größer als 1 ist, kann jedes

Vorkommen von $R^4$ gleich oder verschieden sein und kann am gleichen oder an verschiedenen Ring-Kohlenstoffatomen vorliegen;
$R^5$ ist Wasserstoff, Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen und Hydroxy;
$R^6$ ist Wasserstoff oder Alkyl, enthaltend 1 bis 22 Kohlenstoffatome, Phenyl oder Phenyl, substituiert mit einem oder mehreren Alkyl, enthaltend 1 bis 22 Kohlenstoffatome, Halogen und Hydroxy; oder $R^6$ kann -C(O)O$R^8$ oder -C(O)$R^8$ sein, wobei $R^8$ ist Alkyl, enthaltend 1 bis 22 Kohlenstoffatome; und
$R^7$ ist Phenyl oder Alkyl, enthaltend 1 bis 22 Kohlenstoffatome.

**14.** Verfahren gemäß Anspruch 13, wobei die Peroxydicarbonat- und Diacylperoxidverbindungen der Formel (V):

$$R^9\text{-}(O)_c\text{-}C(O)\text{-}O\text{-}O\text{-}C(O)\text{-}(O)_c\text{-}R^{10} \qquad (V)$$

entsprechen, wobei $R^9$ und $R^{10}$ unabhängig voneinander aliphatische, cycloaliphatische oder aromatische Gruppen, enthaltend 1 bis 22 Kohlenstoffatome, sind, und c ist Null oder Eins.

**15.** Verfahren gemäß Anspruch 14, wobei in Formel (V) beide Indizes c Eins sind.

**16.** Verfahren gemäß Anspruch 14, wobei in Formel (V) beide Indizes c Null sind.

**17.** Verfahren gemäß Anspruch 14, wobei $R^9$ und $R^{10}$ unabhängig voneinander aus der Gruppe, die aus Phenyl, Methyl, Ethyl, n-Propyl, Isopropyl, n-Butyl, sec-Butyl, t-Butyl, Isobutyl, Hexyl, Octyl, Neopentyl, 2-Ethylhexyl, Capryl, Lauryl, Myristyl, Cetyl, Stearyl, Allyl, Methallyl, Crotyl, Cyclohexyl, 4-t-Butylcyclohexyl, 4-t-Amylcyclohexyl, Benzyl, 2-Phenylethyl, 2-Phenylbutyl, α-Carbethoxyethyl, β-Methoxyethyl, 2-Phenoxyethyl, 2-Methoxyphenyl, 3-Methoxyphenyl, 2-Ethoxyethyl, 2-Ethoxyphenyl, 3-Methoxybutyl, 2-Carbamyloxyethyl, 2-Chlorethyl, 2-Nitrobutyl und 2-Nitro-2-methylpropyl besteht, ausgewählt wird.

**18.** Verfahren gemäß Anspruch 13, wobei der Stabilisator aus der Gruppe, die aus Ethyl-2-cyclopentanoncarboxylat, Methyl-2-cycloheptanoncarboxylat, Ethyl-2-cyclohexanoncarboxylat, 2-Acetylcyclopentanon, 2-Acetylcyclohexanon, Ethyl-4-methyl-2-cyclohexanon-1-carboxylat, 1,3-Cyclohexandion, 1,3-Cyclopentandion, 2,4-Pentandion und Dibenzoylmethan, 3-Methyl-1,2-cyclopentandion, 3-Ethyl-1,2-cyclopentandion, 1-2-Cyclohexandion, 1,2-Cyclopentandion und Mischungen davon besteht, ausgewählt wird.

**19.** Verfahren gemäß Anspruch 13, wobei die Menge des Stabilisators 0,2 bis 5 Gew.-% des organischen Peroxids beträgt.

**20.** Verfahren gemäß Anspruch 13, wobei der Stabilisator eine β-Dicarbonylverbindung gemäß Formel (I) umfaßt.

**21.** Verfahren gemäß Anspruch 13, wobei der Stabilisator eine β-Dicarbonylverbindung gemäß Formel (II) umfaßt.

**22.** Verfahren gemäß Anspruch 13, wobei der Stabilisator eine β-Dicarbonylverbindung gemäß Formel (III) umfaßt.

**23.** Verfahren gemäß Anspruch 13, wobei der Stabilisator eine Verbindung gemäß Formel (IV) umfaßt.

**24.** Verfahren gemäß Anspruch 13, wobei die organische Peroxidkomponente aus der Gruppe, die aus Di-2-ethylhexylperoxydicarbonat, Di-sec-butylperoxydicarbonat, Diethylperoxydicarbonat, Di-n-butylperoxydicarbonat, Isopropyl-sec-butylperoxydicarbonat, Di-4-tert-butylcyclohexylperoxydicarbonat, Di-n-propylperoxydicarbonat, Diisopropylperoxydicarbonat, Benzoylperoxid, Dilauroylperoxid, Didecanoylperoxid, Diacetylperoxid, 2-Methylpropionyl-3-methylpentanoylperoxid und Di-(3,5,5-trimethylhexanoyl)peroxid und Mischungen davon besteht, ausgewählt wird.

**Revendications**

**1.** Composition comprenant :

a) un composant peroxyde organique choisi dans le groupe comprenant les composés peroxydicarbonates, les peroxydes de diacyle et leurs mélanges ; et
b) une quantité d'un stabilisant suffisante pour retarder la vitesse de décomposition du composant peroxyde organique, où ledit stabilisant est choisi dans le groupe comprenant les composés β-dicarbonylés ayant les formules I, II et III et les composés de formule IV :

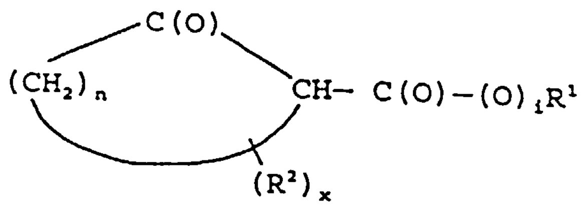
(I)

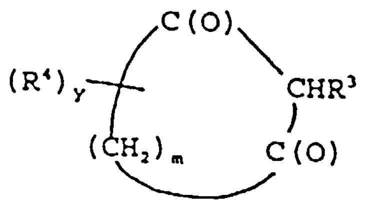
(II)

$$R^5\text{-C(O)-CHR}^6\text{-C(O)-R}^7 \quad \text{(III)}$$

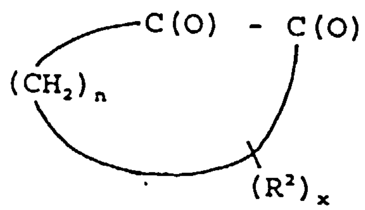
(IV)

et leurs mélanges, où

m vaut 1 - 5,
n vaut 1 - 6,
i vaut 0 - 1,
x vaut 0 - 2n,
y vaut 0 - 2m,
$R^1$ est un groupe alkyle ayant de 1 à 22 atomes de carbone, le groupe phényle, ou un groupe phényle substitué par un ou plusieurs substituants alkyle ayant de 1 à 22 atomes de carbone, halogéno et hydroxy, et $R^1$ peut être un atome d'hydrogène quand i vaut zéro,
$R^2$ est un groupe alkyle ayant de 1 à 22 atomes de carbone, le groupe phényle, ou un groupe phényle substitué par un ou plusieurs substituants alkyle ayant de 1 à 22 atomes de carbone, halogéno et hydroxy, et, quand x est supérieur à 1, tous les radicaux $R^2$ peuvent être identiques ou différents, et ils peuvent se trouver sur des atomes de carbone du cycle identiques ou différents,
$R^3$ est un atome d'hydrogène, un groupe alkyle ayant de 1 à 22 atomes de carbone, acyle ayant de 2 à 22 atomes de carbone, le groupe phényle, ou un groupe phényle substitué par un ou plusieurs substituants alkyle ayant de 1 à 22 atomes de carbone, halogéno et hydroxy ;
$R^4$ est un groupe alkyle ayant de 1 à 22 atomes de carbone, le groupe phényle, ou un groupe phényle substitué par un ou plusieurs substituants alkyle ayant de 1 à 22 atomes de carbone, halogéno et hydroxy ; et quand y est supérieur à 1, tous les radicaux $R^4$ peuvent être identiques ou différents, et peuvent se trouver sur des atomes de carbone du cycle identiques ou différents ;

$R^5$ est un atome d'hydrogène, un groupe alkyle ayant de 1 à 22 atomes de carbone, le groupe phényle, ou un groupe phényle substitué par un ou plusieurs substituants alkyle ayant de 1 à 22 atomes de carbone, halogéno ou hydroxy ;
$R^6$ est un atome d'hydrogène ou un groupe alkyle ayant de 1 à 22 atomes de carbone, le groupe phényle, ou un groupe phényle substitué par un ou plusieurs substituants alkyle ayant de 1 à 22 atomes de carbone, halogéno ou hydroxy ; ou encore $R^6$ peut être -C(O)O$R^8$ ou-C(O)$R^8$ où $R^8$ est un groupe alkyle ayant de 1 à 22 atomes de carbone ; et
$R^7$ est le groupe phényle ou un groupe alkyle ayant de 1 à 22 atomes de carbone.

**2.** Composition selon la revendication 1, comprenant un composé β-dicarbonylé ayant la formule (I) ;

**3.** Composition selon la revendication 1, comprenant un composé β-dicarbonylé ayant la formule (II) ;

**4.** Composition selon la revendication 1, comprenant un composé β-dicarbonylé ayant la formule (III) ;

**5.** Composition selon la revendication 1, comprenant un composé de formule (IV) ;

**6.** Composition selon l'une quelconque des revendications 1 à 5, dans laquelle ledit composant peroxyde organique comprend au moins un composé de formule (V)

$$R^9\text{-}(O)_c\text{-}C(O)\text{-}O\text{-}O\text{-}C(O)(O)_c\text{-}R^{10} \qquad (V)$$

dans laquelle $R^9$ et $R^{10}$ sont chacun indépendamment un groupe aliphatique, cycloaliphatique ou aromatique ayant de 1 à 22 atomes de carbone, et c vaut zéro ou un.

**7.** Composition selon la revendication 6, dans laquelle, dans la formule (V), les deux indices c valent un.

**8.** Composition selon la revendication 6, dans laquelle, dans la formule (V), les deux indices c valent zéro.

**9.** Composition selon la revendication 6, dans laquelle, $R^9$ et $R^{10}$ sont choisis indépendamment dans le groupe comprenant les phényle, méthyle, éthyle, n-propyle, isopropyle, n-butyle, sec-butyle, tert-butyle, isobutyle, hexyle, octyle, néopentyle, 2-éthylhexyle, capryle, lauryle, myristyle, cétyle, stéaryle, allyle, méthallyle, crotyle, cyclohexyle, 4-tert-butylcyclohexyle, 4-tert-amyl-cyclohexyle, benzyle, 2-phényléthyle, 2-phénylbutyle, α-carbéthoxyéthyle, β-méthoxyéthyle, 2-phénoxyéthyle, 2-méthoxy-phényle, 3-méthoxy-phényle, 2-éthoxyéthyle, 2-éthoxyphényle, 3-méthoxybutyle, 2-carbamyloxyéthyle, 2-chloroéthyle, 2-nitrobutyle et 2-nitro-2-méthyl-propyle.

**10.** Composition selon la revendication 1, dans laquelle ledit stabilisant est choisi dans le groupe comprenant le 2-cyclopentanonecarboxylate d'éthyle, le 2-cycloheptanonecarboxylate de méthyle, le 2-cyclohexanonecarboxylate d'éthyle, la 2-acétylcylcopentanone, la 2-acétylcyclohexanone, le 4-méthyl-2-cyclohexanone-1-carboxylate d'éthyle, la 1,3-cyclohexanedione, la 1,3-cyclopentanedione, la 2,4-pentanedione et le dibenzoylméthane, la 3-méthyl-1,2-cyclopentanedione, la 3-éthyl-1,2-cyclopentanedione, la 1,2-cyclohexanedione, la 1,2-cyclopentanedione et leurs mélanges.

**11.** Composition selon la revendication 1, dans laquelle ledit stabilisant représente de 0,2 à 5 % en poids dudit composant peroxyde organique.

**12.** Composition selon la revendication 1, dans laquelle ledit composant peroxyde organique est choisi dans le groupe comprenant le peroxydicarbonate de di-2-éthylhexyle, le peroxydicarbonate de di-sec-butyle, le peroxydicarbonate de diéthyle, le peroxydicarbonate de di-n-butyle, le peroxydicarbonate d'isopropyle-sec-butyle, le peroxydicarbonate de di-4-tert-butylcyclohexyle, le peroxydicarbonate de di-n-propyle, le peroxydicarbonate de di-isopropyle, le peroxyde de benzoyle, le peroxyde de dilauroyle, le peroxyde de didécanoyle, le peroxyde de diacétyle, le peroxyde de 2-méthylpropionyl-3-méthylpentanoyle et le peroxyde de di(3,5,5-triméthylhexanoyle), et leurs mélanges.

**13.** Procédé pour retarder la vitesse de décomposition d'un peroxyde organique choisi dans le groupe comprenant les composés peroxydicarbonates et les composés peroxydes de diacyle et leurs mélanges, comprenant l'addition, audit peroxyde organique, d'un stabilisant en une quantité suffisante pour retarder la vitesse de ladite décomposition, où ledit stabilisant est choisi dans le groupe comprenant les composés β-dicarbonylés ayant les formules I, II et III et les composés de formule IV :

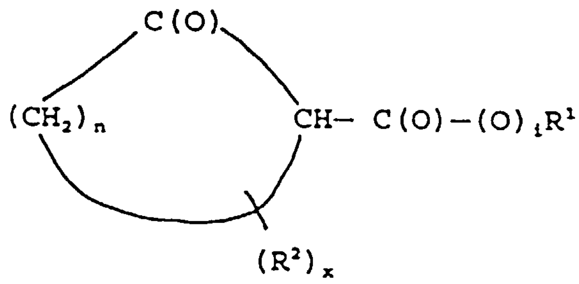

(I)

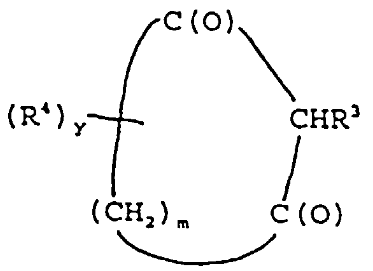

(II)

$$R^5 - C(O) - CHR^6 - C(O) - R^7 \quad (III)$$

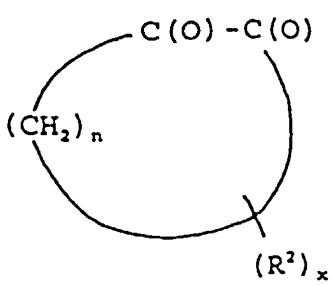

(IV)

et leurs mélanges, où

m vaut 1 - 5,
n vaut 1 - 6,
i vaut 0 - 1,
x vaut 0 - 2n,
y vaut 0 - 2m,
$R^1$ est un groupe alkyle ayant de 1 à 22 atomes de carbone, le groupe phényle, ou un groupe phényle substitué par un ou plusieurs substituants alkyle ayant de 1 à 22 atomes de carbone, halogéno et hydroxy, et $R^1$ peut être un atome d'hydrogène quand i vaut zéro,
$R^2$ est un groupe alkyle ayant de 1 à 22 atomes de carbone, le groupe phényle, ou un groupe phényle substitué par un ou plusieurs substituants alkyle ayant de 1 à 22 atomes de carbone, halogéno et hydroxy, et, quand x est supérieur à 1, tous les radicaux $R^2$ peuvent être identiques ou différents, et ils peuvent se trouver sur des atomes de carbone du cycle identiques ou différents,
$R^3$ est un atome d'hydrogène, un groupe alkyle ayant de 1 à 22 atomes de carbone, acyle ayant de 2 à 22 atomes de carbone, le groupe phényle, ou un groupe phényle substitué par un ou plusieurs substituants alkyle ayant de 1 à 22 atomes de carbone, halogéno et hydroxy ;

$R^4$ est un groupe alkyle ayant de 1 à 22 atomes de carbone, le groupe phényle, ou un groupe phényle substitué par un ou plusieurs substituants alkyle ayant de 1 à 22 atomes de carbone, halogéno et hydroxy ; et quand y est supérieur à 1, tous les radicaux $R^4$ peuvent être identiques ou différents, et peuvent se trouver sur des atomes de carbone du cycle identiques ou différents ;
$R^5$ est un atome d'hydrogène, un groupe alkyle ayant de 1 à 22 atomes de carbone, le groupe phényle, ou un groupe phényle substitué par un ou plusieurs substituants alkyle ayant de 1 à 22 atomes de carbone, halogéno ou hydroxy ;
$R^6$ est un atome d'hydrogène ou un groupe alkyle ayant de 1 à 22 atomes de carbone, le groupe phényle, ou un groupe phényle substitué par un ou plusieurs substituants alkyle ayant de 1 à 22 atomes de carbone, halogéno ou hydroxy ; ou encore $R^6$ peut être -C(O)O$R^8$ ou -C(O)$R^8$ où $R^8$ est un groupe alkyle ayant de 1 à 22 atomes de carbone ; et
$R^7$ est le groupe phényle ou un groupe alkyle ayant de 1 à 22 atomes de carbone.

**14.** Procédé selon la revendication 13, dans lequel lesdits composés peroxydicarbonate et peroxyde de diacyle correspondent à la formule (V) :

$$R^9\text{-(O)}_c\text{-C(O)-O-O-C(O)-(O)}_c\text{-}R^{10} \qquad \text{(V)}$$

dans laquelle $R^9$ et $R^{10}$ sont chacun indépendamment un groupe aliphatique, cycloaliphatique ou aromatique ayant de 1 à 22 atomes de carbone, et c vaut zéro ou un.

**15.** Procédé selon la revendication 14, dans lequel, dans la formule (V), les deux indices c valent un.

**16.** Procédé selon la revendication 14, dans lequel, dans la formule (V), les deux indices c valent zéro.

**17.** Procédé selon la revendication 14, dans lequel $R^9$ et $R^{10}$ sont choisis chacun indépendamment dans le groupe comprenant les phényle, méthyle, éthyle, n-propyle, isopropyle, n-butyle, sec-butyle, tert-butyle, isobutyle, hexyle, octyle, néopentyle, 2-éthylhexyle, capryle, lauryle, myristyle, cétyle, stéaryle, allyle, méthallyle, crotyle, cyclohexyle, 4-tert-butylcyclohexyle, 4-tert-amyl-cyclohexyle, benzyle, 2-phényléthyle, 2-phénylbutyle, α-carbéthoxy-éthyle, β-méthoxyéthyle, 2-phénoxyéthyle, 2-méthoxyphényle, 3-méthoxyphényle, 2-éthoxyéthyle, 2-éthoxyphényle, 3-méthoxybutyle, 2-carbamyloxyéthyle, 2-chloroéthyle, 2-nitrobutyle et 2-nitro-2-méthyl-propyle.

**18.** Procédé selon la revendication 13, dans lequel ledit stabilisant est choisi dans le groupe comprenant le 2-cyclopentanonecarboxylate d'éthyle, le 2-cycloheptanonecarboxylate de méthyle, le 2-cyclohexanonecarboxylate d'éthyle, la 2-acétylcylcopentanone, la 2-acétylcyclohexanone, le 4-méthyl-2-cyclohexanone-1-carboxylate d'éthyle, la 1,3-cyclohexanedione, la 1,3-cyclopentanedione, la 2,4-pentanedione et le dibenzoylméthane, la 3-méthyl-1,2-cyclopentanedione, la 3-éthyl-1,2-cyclopentanedione, la 1,2-cyclohexanedione, la 1,2-cyclopentanedione et leurs mélanges.

**19.** Procédé selon la revendication 13, dans lequel la quantité dudit stabilisant représente de 0,2 à 5 % en poids dudit peroxyde organique.

**20.** Procédé selon la revendication 13, dans lequel ledit stabilisant comprend un composé β-dicarbonylé de formule (I).

**21.** Procédé selon la revendication 13, dans lequel ledit stabilisant comprend un composé β-dicarbonylé de formule (II).

**22.** Procédé selon la revendication 13, dans lequel ledit stabilisant comprend un composé β-dicarbonylé de formule (III).

**23.** Procédé selon la revendication 13, dans lequel ledit stabilisant comprend un composé de formule (IV).

**24.** Procédé selon la revendication 13, dans lequel ledit composant peroxyde organique est choisi dans le groupe comprenant le peroxydicarbonate de di-2-éthylhexyle, le peroxydicarbonate de di-sec-butyle, le peroxydicarbonate de diéthyle, le peroxydicarbonate de di-n-butyle, le peroxydicarbonate d'isopropyle-sec-butyle, le peroxydicarbonate de di-4-tert-butylcyclohexyle, le peroxydicarbonate de di-n-propyle, le peroxydicarbonate de di-isopropyle, le peroxyde de benzoyle, le peroxyde de dilauroyle, le peroxyde de didécanoyle, le peroxyde de diacétyle, le peroxyde

de 2-méthylpropionyl-3-méthylpentanoyle et le peroxyde de di(3,5,5-triméthylhexanoyle), et leurs mélanges.